# EUROPEAN PATENT SPECIFICATION

(11) **EP 4 031 022 B1**
(45) Date of publication and mention of the grant of the patent: **09.12.2026**
(21) Application number: 20781662.0
(22) Date of filing: 14.09.2020
(51) Int. Cl.: A61B 17/11, A61B 17/00, A61F 2/92, A61F 2/95

(54) **LOW-STRESS COMPRESSIBLE IMPLANTS**
SPANNUNGSARME, KOMPRIMIERBARE IMPLANTATE
IMPLANTS COMPRESSIBLES À FAIBLE CONTRAINTE

(30) Priority: 19.09.2019 US 201962902797 P
(43) Date of publication of application: 27.07.2022
(73) Proprietor: Edwards Lifesciences Corporation, Irvine, CA 92614 (US)
(72) Inventor: MAY, Andrew, Charles, Irvine, CA 92614 (US)
(74) Representative: Eisenführ Speiser
(86) International application number: PCT/US2020/050631
(87) International publication number: WO 2021/055266

(56) References cited:
- US-A- 5 618 299
- US-A1- 2003 055 495
- US-A1- 2009 143 713
- US-A1- 2010 268 316
- US-A1- 2016 022 293
- US-A1- 2017 105 839

## Description

### BACKGROUND

### Field

The present invention relates generally to the field of medical devices, in particular to the field of implants.

### Description of Related Art

In percutaneous delivery systems for delivering certain medical implant devices to target locations at least in part through a patient's vasculature, certain anatomical and device dimensions can limit the size, shape, and/or configuration of medical implant devices delivered using such systems.

US 2009/143713 A1 relates to devices, systems, kits and methods for treating biliary disease. Such device comprise, for example, a component configured for deployment between a gallbladder and location within a gastrointestinal tract of a patient which has a proximal end and a distal end with a lumen extending therethrough. A method of deploying the device can be achieved by, for example, creating a duct or fistula between a gallbladder lumen and a portion of a gastrointestinal tract; and providing for drainage from the gallbladder to the gastrointestinal tract.

US 2010/268316 A1 relates to a shunt rivet for implantation between a first body space and a second body space in a patient, such as to treat chronic obstructive pulmonary disease. Further, US 2003/0055495 A1 relates to expandable heart valves for minimally invasive valve replacement surgeries. The valves are rolled into a first, contracted configuration for minimally invasive delivery using a catheter, and then unrolled or unfurled at the implantation site. One- and two-piece stents may be used in conjunction with a plurality of flexible leaflet-forming membranes. The stents may include an annulus section, a sinus section with the membranes attached over sinus apertures, and an outflow section. Lockout tabs and making slots secure the stents in their expanded shapes. Alignment structure ensures concentric unfurling of the stent. Anchoring elements at the stent edges or in the stent body secure the valve within the annulus. Finally, US 5618299 A relates to an intravascular stent including a cylindrical sheet having overlapping edges that interlock. The edges have a series of protrusions and apertures that interlock and ratchet as the stent expands to an open position to support a section of an arterial wall. The stent may be expanded by a balloon catheter or it may be self-expanding.

### SUMMARY

The present invention is defined by the features of the independent claim. Further embodiments are given in the dependent claims. For purposes of summarizing the disclosure, certain aspects, advantages and novel features have been described herein. It is to be understood that not necessarily all such advantages may be achieved in accordance with any particular embodiment. Thus, the disclosed embodiments may be carried out in a manner that achieves or optimizes one advantage or group of advantages as taught herein without necessarily achieving other advantages as may be taught or suggested herein.

In some implementations of the present disclosure, a method comprises rolling a medical implant to reduce a profile of the medical implant. The medical implant comprises a first end and a second end. The method further comprises inserting the medical implant into a catheter, delivering the catheter to a treatment location within a human body, and removing the medical implant from the catheter.

The method may further comprise detaching the first end of the medical implant from the second end of the medical implant prior to rolling the medical implant. In some embodiments, detaching the first end from the second end involves cutting the medical implant. Detaching the first end from the second end may involve disengaging an attachment mechanism at the first end. In some embodiments, the method further comprises attaching the first end to the second end after removing the medical implant from the catheter. Attaching the first end to the second end may involve engaging an attachment mechanism at the first end.

In some embodiments, removing the medical implant from the catheter causes unrolling of the medical implant to an expanded profile. A width of the medical implant in the expanded profile may exceed a width of the catheter. In some embodiments, the method further comprises unrolling the medical implant to an expanded profile. A width of the medical implant in the expanded profile may exceed a width of the catheter. In some embodiments, rolling the medical implant causes at least some overlap between the first end and the second end. Rolling the medical implant may cause no overlap between the first end and the second end.

The medical implant may naturally assume a generally flat form. In some embodiments, the medical implant is at least partially composed of Nitinol. The medical implant may comprise an elongate body and one or more anchoring arms. In some embodiments, the one or more anchoring arms are configured to extend perpendicularly from the elongate body. The elongate body and the one or more anchoring arms may be configured to be rolled.

The present invention relates to a medical implant comprising an elongate body having a first end and a second end. The elongate body is configured to be rolled to a reduced profile and fit into a catheter in the reduced profile.

The elongate body is further configured to unroll to an expanded profile in response to being removed from the catheter. The medical implant may further comprise one or more attachment mechanisms configured to attach the first end to the second end in the expanded profile of the elongate body. The medical implant further comprises one or more anchoring arms extending from the elongate body. The one or more anchoring arms are configured to anchor to one or more tissue walls.

In some embodiments, the elongate body is further configured to prevent in-growth of tissue through the elongate body. The elongate body may be configured to expand in response to expansion of a tissue wall. In some embodiments, the elongate body is configured to fit at least partially within an opening in a tissue wall. The tissue wall may be situated between a first anatomical chamber and a second anatomical chamber and the opening may represent a blood flow path between the first anatomical chamber to the second anatomical chamber. In some embodiments, the elongate body is configured to maintain the blood flow path from the first anatomical chamber to the second anatomical chamber.

Some implementations of the present disclosure relate to a medical implant comprising a central flow portion configured to define a flow path between two anatomical chambers of a heart. The central flow portion comprises a first end and a second end. The first end comprises one or more attachment mechanisms configured to alternately join with and detach from the second end. Joining the first end to the second end shapes the central flow portion to a generally tubular form. The central flow portion is configured to be rolled when the first end is detached from the second end. The medical implant further comprises two or more anchoring arms configured to extend from the central flow portion and anchor to a tissue wall separating the two anatomical chambers.

Rolling the central flow portion may cause at least partial overlap of the central flow portion. In some embodiments, rolling the central flow portion causes no overlap of the central flow portion and increases a distance between the first end and the second end. The two or more anchoring arms may be configured to be rolled. In some embodiments, the central flow portion is configured to be inserted into a catheter after being rolled and naturally unroll in response to being removed from the catheter.

### BRIEF DESCRIPTION OF THE DRAWINGS

Various embodiments are depicted in the accompanying drawings for illustrative purposes and should in no way be interpreted as limiting the scope of the invention. In addition, various features of different disclosed embodiments can be combined to form additional embodiments, which are part of this disclosure. Throughout the drawings, reference numbers may be reused to indicate correspondence between reference elements. However, it should be understood that the use of similar reference numbers in connection with multiple drawings does not necessarily imply similarity between respective embodiments associated therewith. Furthermore, it should be understood that the features of the respective drawings are not necessarily drawn to scale, and the illustrated sizes thereof are presented for the purpose of illustration of inventive aspects thereof. Generally, certain of the illustrated features may be relatively smaller than as illustrated in some embodiments or configurations.
Figure 1 illustrates several access pathways for maneuvering guidewires and/or catheters in and around the heart to deploy compressible implants in accordance with some embodiments.
Figure 2 depicts a method for deploying implants in accordance with some embodiments.
Figures 3A and 3B illustrate components of delivery systems for delivering one or more frames in accordance with one or more embodiments.
Figures 4A-4C illustrate side views of compression stages of a compressible frame in accordance with some embodiments.
Figures 5A and 5B show multiple spiral compression stages of an example frame shown from above in accordance with some embodiments, not forming part of the claimed invention.
Figures 6A-6C illustrate a compressible frame in accordance with some embodiments.
Figures 7-1 and 7-2 are a flow diagram illustrating a process for rolling, spiraling, and/or twisting a frame to minimize the profile of the frame during delivery to a treatment site in accordance with one or more embodiments of the present disclosure.
Figures 8-1 and 8-2 show an example frame associated with the process of Figures 7-1 and 7-2 to illustrate aspects of the process according to one or more implementations thereof.

### DETAILED DESCRIPTION

The headings provided herein are for convenience only and do not necessarily affect the scope or meaning of the claimed invention.

Heart failure is a common and potentially lethal condition affecting humans, with sub-optimal clinical outcomes often resulting in symptoms, morbidity and/or mortality, despite maximal medical treatment. In particular, "diastolic heart failure" refers to the clinical syndrome of heart failure occurring in the context of preserved left ventricular systolic function (ejection fraction) and in the absence of major valvular disease. This condition is characterized by a stiff left ventricle with decreased compliance and impaired relaxation, which leads to increased end-diastolic pressure. Approximately one third of patients with heart failure have diastolic heart failure and there are very few, if any, proven effective treatments.

Symptoms of diastolic heart failure are due, at least in part, to an elevation in pressure in the left atrium. Elevated Left Atrial Pressure (LAP) is present in several abnormal heart conditions, including Heart Failure (HF). In addition to diastolic heart failure, a number of other medical conditions, including systolic dysfunction of the left ventricle and valve disease, can lead to elevated pressures in the left atrium. Both Heart Failure with Preserved Ejection Fraction (HFpEF) and Heart Failure with Reduced Ejection Fraction (HFrEF) can exhibit elevated LAP and may benefit from a reduction in LAP, which can in turn reduce the systolic preload on the left ventricle, Left Ventricular End Diastolic Pressure (LVEDP). It can also relieve pressure on the pulmonary circulation, reducing the risk of pulmonary edema, improving respiration and improving patient comfort.

The following includes a general description of human cardiac anatomy that is relevant to certain inventive features and embodiments disclosed herein and is included to provide context for certain aspects of the present disclosure. In humans and other vertebrate animals, the heart is a hollow muscular organ having four pumping chambers: the left and right atria and the left and right ventricles, each provided with its own one-way valve. The natural heart valves are identified as the aortic, mitral (or bicuspid), tricuspid and pulmonary, and are each mounted in an annulus comprising dense fibrous rings attached either directly or indirectly to the atrial and ventricular muscle fibers. Each annulus defines a flow orifice. The four valves ensure that blood does not flow in the wrong direction during the cardiac cycle; that is, to ensure that the blood does not back flow through the valve. Blood flows from the venous system and right atrium through the tricuspid valve to the right ventricle, then from the right ventricle through the pulmonary valve to the pulmonary artery and the lungs. Oxygenated blood then flows through the mitral valve from the left atrium to the left ventricle, and finally from the left ventricle through the aortic valve to the aorta/arterial system.

Heart failure is a common and potentially lethal condition affecting humans, with sub-optimal clinical outcomes often resulting in symptoms, morbidity and/or mortality, despite maximal medical treatment. In particular, "diastolic heart failure" refers to the clinical syndrome of heart failure occurring in the context of preserved left ventricular systolic function (ejection fraction) and in the absence of major valvular disease. This condition is characterized by a stiff left ventricle with decreased compliance and impaired relaxation, which leads to increased end-diastolic pressure. Approximately one third of patients with heart failure have diastolic heart failure and there are very few, if any, proven effective treatments.

Symptoms of diastolic heart failure are due, at least in a large part, to an elevation in pressure in the left atrium. Elevated Left Atrial Pressure (LAP) is present in several abnormal heart conditions, including Heart Failure (HF). In addition to diastolic heart failure, a number of other medical conditions, including systolic dysfunction of the left ventricle and valve disease, can lead to elevated pressures in the left atrium. Both Heart Failure with Preserved Ejection Fraction (HFpEF) and Heart Failure with Reduced Ejection Fraction (HFrEF) can exhibit elevated LAP. It has been hypothesized that both subgroups of HF might benefit from a reduction in LAP, which in turn reduces the systolic preload on the left ventricle, Left Ventricular End Diastolic Pressure (LVEDP). It could also relieve pressure on the pulmonary circulation, reducing the risk of pulmonary edema, improving respiration and improving patient comfort.

Pulmonary hypertension (PH) is defined as a rise in mean pressure in the main pulmonary artery. PH may arise from many different causes, but, in all patients, has been shown to increase mortality rate. A deadly form of PH arises in the very small branches of the pulmonary arteries and is known as Pulmonary Arterial Hypertension (PAH). In PAH, the cells inside the small arteries multiply due to injury or disease, decreasing the area inside of the artery and thickening the arterial wall. As a result, these small pulmonary arteries narrow and stiffen, causing blood flow to become restricted and upstream pressures to rise. This increase in pressure in the main pulmonary artery is the common connection between all forms of PH regardless of underlying cause. Despite previous attempts, there is a need for an improved way to reduce elevated pressure in the left atrium, as well as other susceptible heart chambers such as the pulmonary artery.

The present disclosure provides methods and devices for delivering implants and/or similar devices to desired locations within a human body. The terms "implant" and/or "means for treatment" may be used herein according to their plain and ordinary meaning and may refer to any medical implant, frame, valve, shunt, stent, anchor, and/or similar devices for use in treating various conditions in a human body. Implants may be delivered via catheter (i.e., transcatheter) for various medical procedures and may have a generally sturdy and/or flexible structure. The terms "catheter," "sheath," and/or "means for delivery" may be used herein according to their broad and ordinary meaning and may include any tube, sheath, steerable sheath, steerable catheters, and/or any other type of elongate tubular delivery device comprising an inner lumen configured to slidably receive instrumentation, such as for positioning within an atrium or coronary sinus, including for example delivery catheters and/or cannulas. In some cases, an implant may be at least partially composed of a shape-memory alloy (e.g., Nitinol) and/or may have a pre-defined shape and/or structure. The implant may be configured to be shaped and/or compressed to fit into a catheter. In some cases, an implant may at least partially have an elliptical and/or cylindrical form and/or may comprise an interweaving pattern of materials.

Conventional designs and/or related compression methods of implants (e.g., Nitinol implants) may have a variety of limitations. For example, some implants may be compressible to a given profile that may not allow the implant to fit into some catheters. The term "profile" is used herein according to its plain and ordinary meaning and may refer to one of and/or a combination of a width, surface area, diameter, radius, length, height, depth, and/or other measurement of a device and/or object. Moreover, size reduction using certain compression methods, including crimping and/or sheathing, may create stresses within the implant. When the implant is compressed beyond certain limits for the implant, the implant may be fractured and/or permanently deformed. For example, a Nitinol implant may be configured to have a pre-defined shape but may be crimped to form a smaller profile to fit into a catheter. When the implant is removed from the catheter, the implant may return to the pre-defined shape unless the compression process fractured and/or deformed the implant to an extent that prevents the implant from naturally returning to the pre-defined shape. Accordingly, the compression profile for the implant may be limited to a size at which permanent deformation of the implant is prevented. While such a compression profile may prevent the implant from being compatible with certain catheters, the malleability of the implant may advantageously allow the implant to be usable with a variety of catheters and may simplify delivery processes for surgeons.

Some embodiments of the present disclosure provide implants and/or compression methods for implants with minimal stress and/or strain to the material of the implants. In some embodiments, an implant may be rolled and/or otherwise compressed to achieve a relatively small profile with respect to conventional methods and/or generally equal profile with relatively lower material strain than conventional methods. The terms "roll," "rolling," "rolled," etc. are used herein according to their broad and ordinary meaning and may refer to any method of at least partially bending and/or curving a device, and may include twisting, spiraling, and/or other methods. In some cases, rolling a medical implant may cause portions and/or multiple ends of the implant to at least partially overlap at least at one stage of the rolling process. In other cases, rolling (e.g., spiraling) may cause no overlap of multiple end of the implant. For example, an implant may be spiraled such that a distance between multiple ends increases as the implant is rolled and/or sections of the implant may not overlap and/or may minimally overlap. an implant may be configured to be cut and/or may comprise a non-continuous form such that the implant may comprise multiple ends configured to move independently and/or be disconnected in a compressed form of the implant.

In some embodiments, an implant may comprise one or more generally flat components and/or one or more components having a generally flat pre-defined shape. Regardless of the pre-defined shape of the implant, the implant may be configured to be rolled and/or otherwise form an elliptical and/or tubular shape to approximate a shape of a delivery catheter configured to receive the implant. The implant may further comprise various anchoring elements, means for anchoring, and/or other features configured to anchor the implant to tissue after delivery via a catheter.

An implant may be gradually compressed from an uncompressed form to a compressed form and/or may be compressed in multiple stages. In the uncompressed form, the implant may have a larger profile than a delivery catheter and/or an inner lumen of the delivery catheter. The implant may be compressed until the profile of the implant is approximately equal to or less than a size of the delivery catheter and/or the inner lumen of the delivery catheter. When the implant is removed from the catheter, the implant may naturally return to the uncompressed form and/or expanded profile and/or may be manually expanded and/or assisted in expansion to any form through various means. In some embodiments, the implant may comprise one or more tabs, clips, clasps, hooks, loops, or other devices or mechanisms configured to hold or lock the implant in a compressed and/or uncompressed form. For example, the implant may comprise interlocking tabs configured to join multiple disconnected ends of the implant together. However, an implant may not necessarily include locking mechanisms and/or may not necessarily be used in conjunction with locking mechanisms. Details of these methods, implants and deployment systems will be described below.

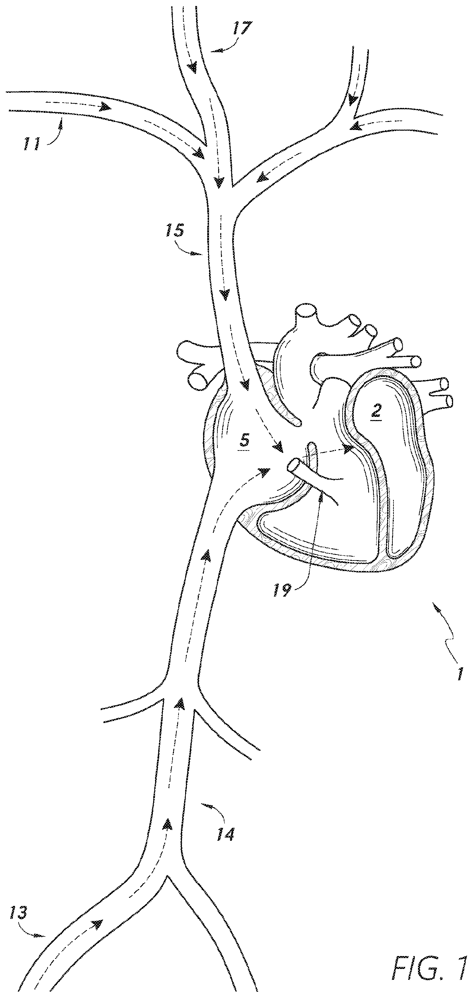

Figure 1 illustrates several access pathways for maneuvering guidewires and catheters in and around the heart 1 to deploy compressible medical implants (e.g., frames) of the present application. For instance, access may be from above via either the subclavian vein 11 or jugular vein 17 into the superior vena cava (SVC) 15, right atrium (RA) 5 and from there into the coronary sinus (CS) 19. Alternatively, the access path may start in the femoral vein 13 and through the inferior vena cava (IVC) 14 into the heart 1. Other access routes may also be used, and each typically utilizes a percutaneous incision through which the guidewire and catheter are inserted into the vasculature, normally through a sealed introducer, and from there the physician controls the distal ends of the devices from outside the body.

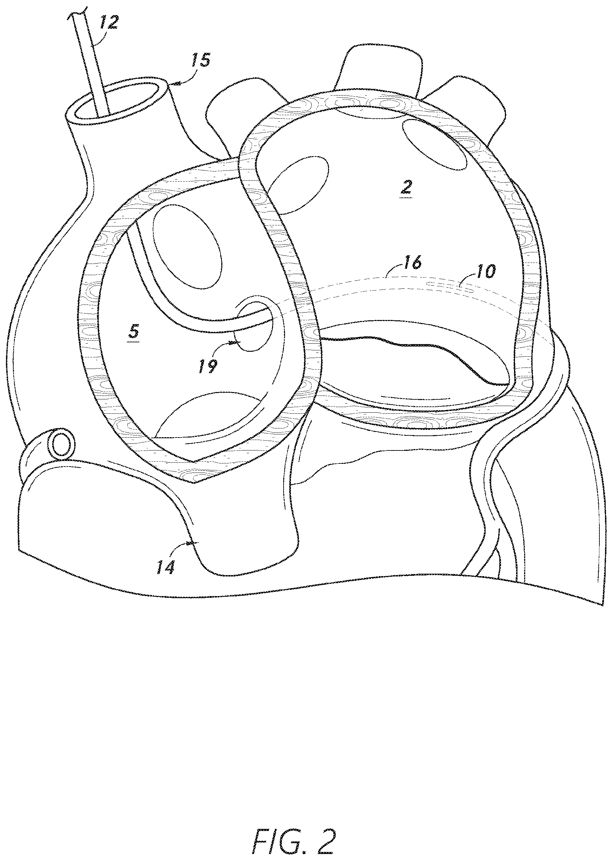

Figure 2 depicts an example method for deploying the medical implants 10 described herein, wherein a guidewire 16 is introduced through the subclavian or jugular vein, through the SVC 15 and into the coronary sinus 19. Once the guidewire 16 provides a path, an introducer sheath (not shown) may be routed along the guidewire 16 and into the patient's vasculature, typically with the use of a dilator. Figure 2 shows a deployment catheter 12 extending from the SVC 15 to the coronary sinus 19 of the heart 1, the deployment catheter 12 having been passed through the introducer sheath which provides a hemostatic valve to prevent blood loss.

In one embodiment, the deployment catheter 12 may be about 30 cm long, and the guidewire 16 may be somewhat longer for ease of use. In some embodiments, the deployment catheter 12 may function to form and prepare an opening in the wall of the left atrium 2, and a separate placement or delivery catheter will be used for delivery of an expandable implant 10. In other embodiments, the deployment catheter 12 may be used as both the puncture preparation and implant placement catheter with full functionality. In the present application, the terms "deployment catheter" or "delivery catheter" will be used to represent a catheter 12 or introducer with one or both of these functions.

Since the coronary sinus 19 is largely contiguous around the left atrium 2, there are a variety of possible acceptable placements for implants 10. The site selected for placement of the implant 10 (e.g., stent), may be made in an area where the tissue of the particular patient is less thick or less dense, as determined beforehand by non-invasive diagnostic means, such as a CT scan or radiographic technique, such as fluoroscopy or intravascular coronary echo (IVUS).

Some methods to reduce LAP involve utilizing an implant 10 between the left atrium 2 and the right atrium 5, through the interatrial septum therebetween. This is a convenient approach, as the two structures are adjacent and transseptal access is common practice. However, there may be a possibility of emboli travelling from the right side of the heart to the left, which presents a stroke risk. This event should only happen if the right atrium pressures go above left atrium pressures; primarily during discrete events like coughing, sneezing, Valsalva maneuver, or bowel movements. The anatomical position of the septum would naturally allow emboli to travel freely between the atria if an implant 10 was present and the pressure gradient flipped. This can be mitigated by a valve or filter element in the implant 10, but there may still be risk that emboli will cross over.

Implanting to the coronary sinus 19 offers some distinct advantages, primarily that the coronary sinus 19 is much less likely to have emboli present for several reasons. First, the blood draining from the coronary vasculature into the right atrium 5 has just passed through capillaries, so it is essentially filtered blood. Second, the ostium of the coronary sinus 19 in the right atrium 5 is often partially covered by a pseudo-valve called the Thebesian Valve. The Thebesian Valve is not always present, but some studies show it is present in more than 60% of hearts and it would act as a natural "guard dog" to the coronary sinus to prevent emboli from entering in the event of a spike in right atrium pressure. Third, a pressure gradient between the coronary sinus 19 and the right atrium 5 into which it drains can be very low, meaning that emboli in the right atrium 5 is likely to remain there. Fourth, in the event that emboli do enter the coronary sinus 19, there may be a much greater gradient between the right atrium 5 and the coronary vasculature than between the right atrium 5 and the left atrium 2. Most likely, emboli may travel further down the coronary vasculature until right atrium pressure returns to normal and then the emboli may return directly to the right atrium 5.

Some additional advantages to locating the implant 10 between the left atrium 2 and the coronary sinus 19 is that this anatomy is less mobile than the septum (e.g., it is more stable) and thus preserves the septum for later transseptal access for alternate therapies, and it could potentially have other therapeutic benefits. By diverting left atrial blood into the coronary sinus 19 (e.g., using a Neovasc Reducer), sinus pressures may increase by a small amount. This can cause blood in the coronary vasculature to travel more slowly through the heart, increasing perfusion and oxygen transfer, which can be more efficient and also can help a dying heart muscle to recover. The preservation of transseptal access can also provide a significant advantage because HF patients often have a number of other comorbidities (e.g., Atrial Fibrillation (AF) and Mitral Regurgitation (MR)) and several of the therapies for treating these conditions require a transseptal approach.

An implant 10 may also be positioned within chambers and/or vessels and/or between other cardiac chambers, such as between the pulmonary artery and right atrium 5. The implant 10 may be desirably implanted within the wall of the pulmonary artery using the deployment tools described herein, with the catheters 12 approaching from above and passing through the pulmonary artery. As explained above, pulmonary hypertension (PH) is defined as a rise in mean pressure in the main pulmonary artery. Blood flows through the implant 10 from the pulmonary artery into the right atrium 5 if the pressure differential causes flow in that direction, which attenuates pressure and reduces damage to the pulmonary artery. The purpose is to attenuate pressure spikes in the pulmonary artery. The implant may also extend from the pulmonary artery to other heart chambers (e.g., left atrium 2) and/or blood vessels. In some embodiments, the implant 10 may further contain a one-way valve for preventing backflow, or a check valve for allowing blood to pass only above a designated pressure. Some implants 10 described herein may be at least partially compressible and/or expandable. Moreover, in some embodiments, an implant 10 may have various features and/or may be used in combination with devices having various barriers for preventing, inhibiting, and/or containing tissue growth. However, implants 10 may not necessarily be used in conjunction with other devices designed to impact tissue growth. The implant 10 may be configured to at least partially prevent, inhibit, reduce, contain, and/or otherwise alter tissue growth and/or in-growth of tissue at and/or around the implant 10 and/or within an opening in a tissue wall. Implants 10 described herein may have various features to simplify and/or improve delivery procedures for surgeons. For example, an implant 10 may be at least partially flexible, compressible, and/or elastic to allow the implant 10 to be shaped and/or molded as necessary/desired to fit into delivery catheters 12 having various sizes and/or shapes. Moreover, an implant 10 may be configured to fit within and/or pass through various openings created in tissue walls having various sizes and/or shapes. A tissue wall may be situated between a first anatomical chamber (e.g., the coronary sinus 19) and a second anatomical chamber (e.g., the left atrium 2). In some embodiments, an opening may be created through the tissue wall and/or the implant 10 (e.g., an elongate body and/or central flow portion of the implant 10) may be configured to fit at least partially within the opening. The opening may represent a blood flow path between the first anatomical chamber and the second anatomical chamber. In some embodiments, the implant 10 may be configured to maintain the opening and/or the blood flow path from the first anatomical chamber to the second anatomical chamber.

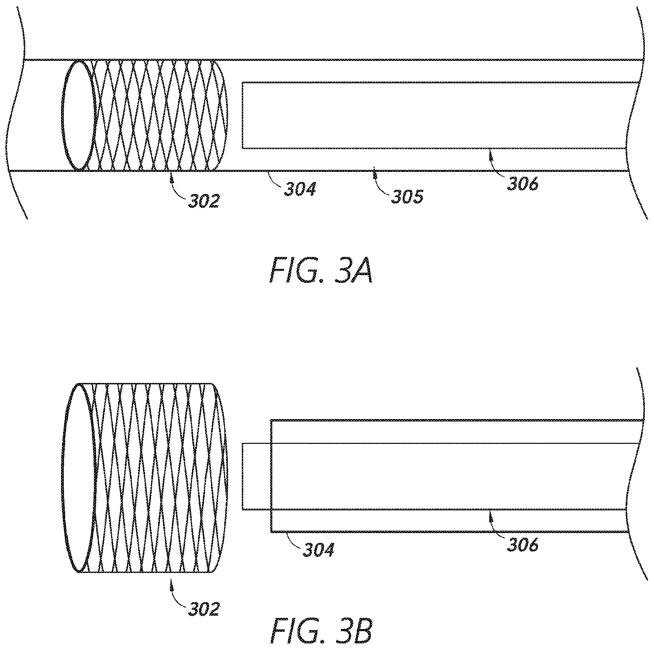

Figures 3A and 3B illustrate components of delivery systems for delivering one or more implants in accordance with one or more embodiments. Figure 3A illustrates a delivery (e.g., closed) state of the delivery systems, in which an implant 302 may be situated within an inner lumen 305 of a catheter 304. At delivery, the implant 302 may be in a compressed form and/or may have a reduced and/or minimal profile. For example, an inner wall of the catheter 304 may press against the implant 302 to prevent the implant 302 from expanding to an expanded profile (shown in Figure 3B).

In some embodiments, the catheter 304 may pass through and/or attach to a handle and/or other delivery mechanism which may be situated outside the body. An inner support shaft 306 may attach to the handle and/or to the catheter 304 to provide support to the handle and/or catheter 304. The inner support shaft 306 may be situated at least partially within the inner lumen 305. In some embodiments, the catheter 304 may be controllable. For example, the catheter 304 may be extended and/or pulled back with respect to the implant 302. In some embodiments, the implant 302 may be delivered without use of a support shaft 306. The implant 302 may comprise one or more wires, struts, and/or other components which may interconnect and/or overlap to form a mesh and/or web-like pattern with one or more gaps/openings (i.e., cells) between the various components, as shown in Figures 3A and 3B.

Figure 3B illustrates an open state of the delivery systems. As shown in Figure 3B, the catheter 304 may be pulled back and/or the implant 302 may be extended to expose the implant 302 outside of the catheter 304. For example, during the delivery stage (shown in Figure 3A), the implant 302 may at least partially be in contact with the inner surface of the catheter 304. When the catheter 304 is pulled back, the implant 302 may not be in contact with the inner surface of the catheter 304. In some embodiments, the implant 302 may be configured to expand when the implant 302 is exposed. For example, the implant 302 may be at least partially composed of a shape-memory alloy (e.g., Nitinol) and may be configured to naturally assume a pre-defined shape. In some embodiments, the implant 302 may be configured to be manually shaped and/or otherwise assisted in expanding after exiting the catheter 304.

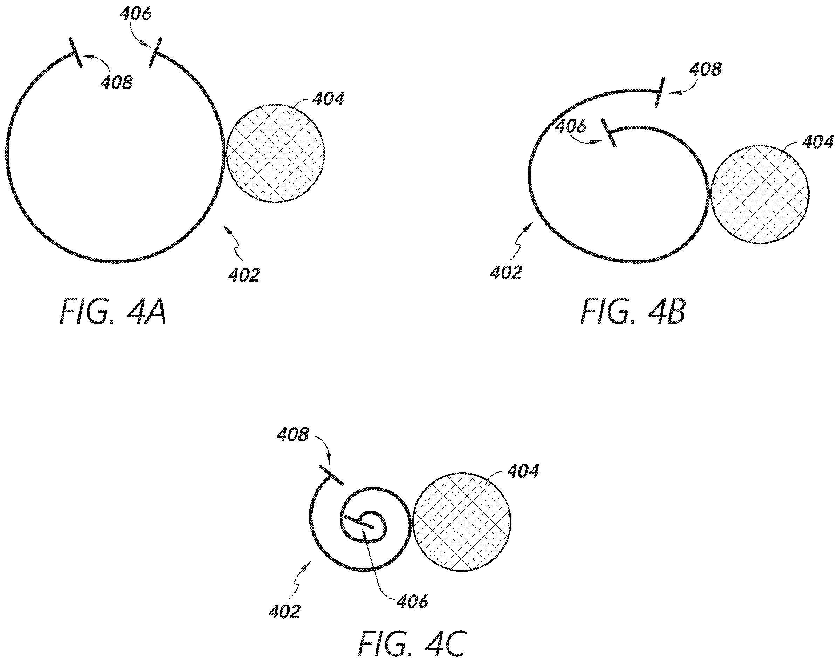

Figures 4A-4C illustrate side views of compression stages of a compressible implant 402 in accordance with some embodiments. The implant 402 is shown with respect to a side view of an opening of a catheter 404 which may be configured to receive the implant 402 when the implant 402 is in a fully compressed state (shown in Figure 4C). Figure 4A shows a first stage (e.g., an un-compressed and/or a first compression stage) of the implant 402. While the implant 402 is represented as a line in Figures 4A-4C, the implant 402 may comprise a network of one or more wires, struts, and/or other features forming cells and/or other features (see, e.g., the implant 302 of Figure 3A and Figure 3B).

In some embodiments, the implant 402 may be configured to form an elliptical and/or circular shape during one or more compression stages. As shown in Figure 4A, the implant 402 may have a much larger radius and/or width than the catheter 404 at the first stage. Accordingly, the implant 402 may not fit into the catheter 404 at the first stage.

While the implant 402 is shown having an elliptical form at the first stage (in Figure 4A), the implant 402 may have a non-elliptical pre-defined form and/or may not have any pre-defined form and/or may be shaped to a desired form. For example, the implant 402 may have a generally flat form. The implant 402 may be at least partially composed of a shape-memory alloy (e.g., Nitinol) and may be configured to be rolled and/or otherwise shaped to form an elliptical shape as shown in Figures 4A-4C.

In some embodiments, the implant 402 may comprise multiple ends, including a first end 406 and a second end 408. The multiple ends may be formed by cutting and/or otherwise creating a separation in the implant 402. The implant 402 may have a pre-defined form in which the first end 406 and the second end 408 may be in contact and/or in close proximity to each other in the pre-defined form, as shown in Figure 4A. For example, the implant 402 may comprise a generally elliptical structure. When the implant 402 is cut, the implant 402 may comprise the first end 406 and the second end 408. Due at least in part to the pre-defined form of the implant 402, the first end 406 and the second end 408 may remain in close proximity and/or in contact with each other after the implant 402 is cut.

Rather than being configured to be cut, the implant 402 may have a non-continuous form in which the implant 402 comprises at least the first end 406 and the second end 408. The implant 402 comprises one or more attaching and/or locking mechanisms configured to join the first end 406 and the second end 408. For example, the first end 406 and/or the second end 408 may comprise one or more tabs, hooks, clasps, pins, loops, and/or other features configured interact with corresponding features at the second end 408 and/or first end 406. For example, the first end 406 may comprise one or more tabs configured to interlock with corresponding tabs and/or fit into corresponding cavities at the second end 408.

Figure 4B shows an intermediate compression stage of the implant 402. As shown in Figure 4B, a diameter and/or width of the implant 402 at the intermediate stage may be greater than a diameter and/or width of the catheter 404. In some embodiments, a general shape and/or form of the implant 402 may remain consistent through all compression stages (i.e., stages shown in Figures 4A-4C). In other words, the shape of the implant 402 at the intermediate stage (shown in Figure 4B) may be similar to the shape of the implant 402 at the first stage (shown in Figure 4A) and/or at a final compression stage (shown in Figure 4C). For example, the implant 402 may maintain an elliptical form (at least when viewed from the side as in Figures 4A-4C) through the first stage, intermediate stage, and final stage. In this way, compression of the implant 402 may be approximately equal at different parts of the implant 402. For example, as the implant 402 compresses, each part of the implant 402 may bend, curve, twist, stretch, and/or otherwise compress approximately equally such that no portions of the implant 402 are required to have more than a minimal amount of deformation. Thus, the levels of stress and/or likelihood of damage at the implant 402 may be minimized.

At the intermediate stage, the first end 406 and the second end 408 may be displaced from each other. In some embodiments, the implant 402 may be configured to be rolled. Accordingly, portions of the implant 402 may be configured to overlap with each other (e.g., form coils) as the implant 402 compresses. Moreover, as the implant 402 compresses, the first end 406 may be situated under at least some portions of the implant 402 and/or may overlap at least partially with the second end 408. In other words, the first end may be nearer (relative to the second end 408) a center point about which the implant 402 is rolled. The second end 408 may be configured to be situated over at least some portions of the implant 402 (e.g., further from the center point 410 than the first end 406).

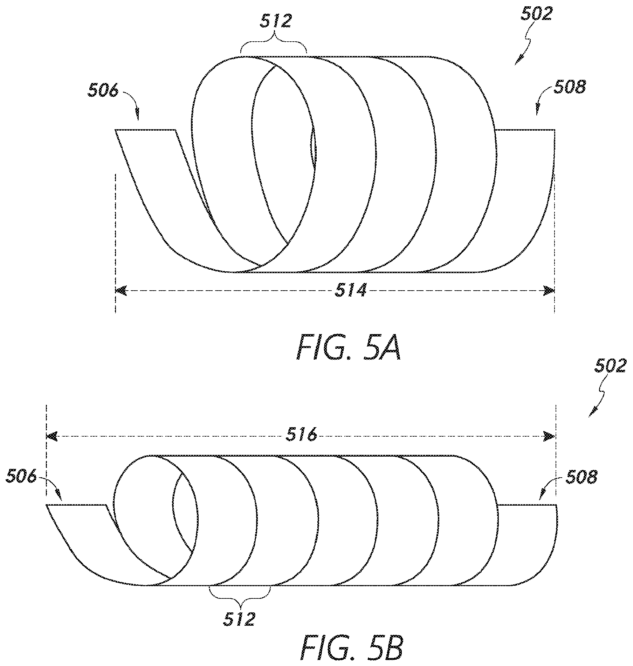

In some embodiments, the implant 402 may be configured to be spiraled (see, e.g., Figures 5A and 5B). Accordingly, the implant 402 may be configured to expand laterally during the compression process and/or there may be little or no overlap at the implant 402 and/or little or no overlap between the first end 406 and the second end 408. As the implant 402 is compressed, the diameter and/or width of the implant 402 may decrease as a length (e.g., a distance between the first end 406 and the second end 408) increases.

Figure 4C shows the final compression stage of the implant 402. At the final stage, the implant 402 may have a diameter and/or width that is approximately equal to or less than a diameter and/or width of the catheter 404. The implant 402 may have a generally elliptical form at the final stage. When the implant 402 reaches the final compression stage, the level of stress at one or more points of the implant 402 may be at a maximum amount. For example, stress levels at one or more points of the implant 402 may gradually increase as the implant 402 moves towards the final stage. In some embodiments, the implant 402 may be configured and/or may be compressed such that stress levels at the implant are generally equal throughout all portions of the implant 402. For example, as the implant 402 is rolled and/or spiraled, all points of the implant 402 may be bent, twisted, curved, and/or otherwise shaped at an approximately equal pace so as to spread out the stress along the length of the implant 402.

In some embodiments, the implant 402 may be configured to be held in the final compression form shown in Figure 4C by the catheter 404. For example, at the final stage, the implant 402 may be inserted into the catheter 404 and the catheter 404 may be configured to prevent the implant 402 from expanding. In some embodiments, the implant 402 may comprise one or more locking mechanisms configured to hold the implant 402 in a compressed position (e.g., in the final compression stage form). For example, the implant 402 may comprise one or more tabs configured to interact with other portions of the implant 402 to prevent expansion of the implant 402. In this way, following compression of the implant 402, a surgeon may more easily place the implant 402 into the catheter 404.

At the final compression stage (shown, e.g., in Figure 4C), the implant 402 may have a maximal amount of overlap. For example, as the implant 402 compresses, an amount of overlap of the implant 402 may gradually increase until the implant 402 reaches maximal overlap and/or compression at the final compression stage. The second end 408 may be at an outer portion of the implant 402 and/or the first end 406 may be at an inner portion of the implant 402. In embodiments in which the implant 402 is spiraled, the implant 402 may have a maximal length and/or a minimal diameter/width at the final compression stage. The compressibility and/or malleability of the implant 402 may advantageously allow surgeons to deliver the implant 402 percutaneously rather than surgically and/or with use of any of a variety of catheters 404.

Figures 5A and 5B show multiple spiral compression stages of an example implant 502 shown from above in accordance with some embodiments. Figure 5A shows a first stage and/or an intermediate stage of the implant 502. In other words, the implant 502 may be uncompressed, minimally compressed, and/or at least partially compressed in Figure 5A. The implant 502 comprises a first end 506 and a second end 508. The implant 502 may have a pre-defined elliptical and/or cylindrical form and/or may be compressed to form an elliptical and/or cylindrical form. For example, as shown in Figure 5A, the implant 502 may form an outer tube around a hollow inner portion having a first diameter. The implant 502 may comprise a first length 514 at the first stage and/or intermediate stage.

As the implant 502 is spiraled, the implant 502 may form one or more coils 512, which may represent adjoined portions of the implant 502. The number of coils 512 may increase as the implant 502 compresses. By creating a spiral and/or coiled form during the compression stages, the implant 502 may effectively distribute stress at the implant 502 evenly across the length of the implant 502. For example, as the implant 502 compresses, all portions of the implant 502 may have an approximately equal amount of compression from a pre-defined form. The implant 502 may form a tubular coil in which all portions of the implant 502 have an approximately equal distance form a center point of the implant 502. In this way, risk of deformation of the implant 502 may be minimized.

Figure 5B shows a second compression stage and/or final compression stage of the implant 502. The implant 502 may comprise a second length 516 at the second and/or final compression stage. The second length 516 may be greater than the first length 514. Moreover, the implant 502 may have a second diameter at the second and/or final compression stage. The second diameter may be smaller than the first diameter. The implant 502 may further comprise a greater number of coils 512 at the second and/or final compression stage than at the first and/or intermediate stage.

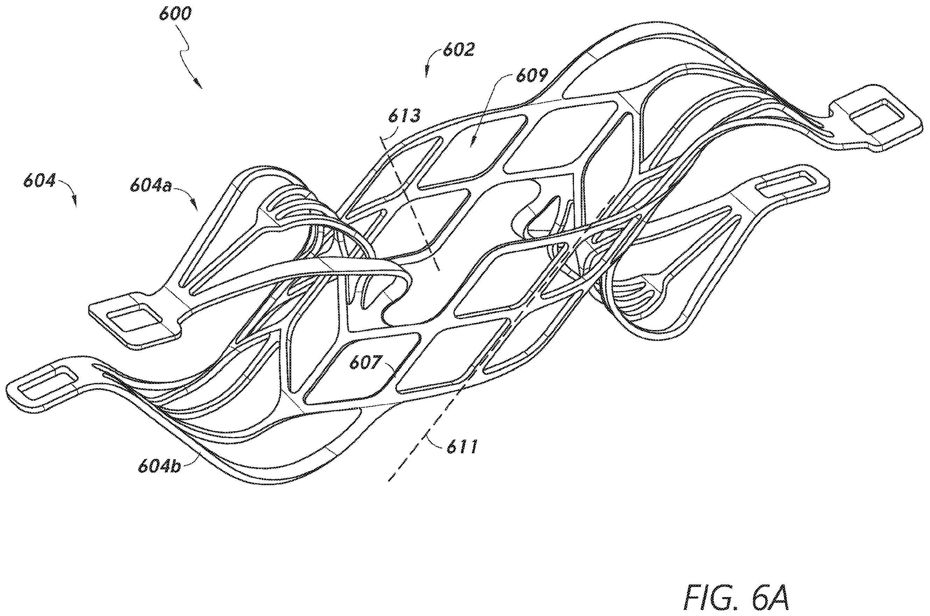

Figures 6A-6C illustrate a compressible implant 600 in accordance with some embodiments. The implant 600 may comprise any of a variety of features and/or components configured to treat various medical conditions. For example, an implant 600 may be configured to maintain an opening in a tissue wall and/or allow blood flow through the tissue wall. In some embodiments, the implant 600 may comprise an elongate body 602 which may be configured to be situated at least partially within an opening in a tissue wall. The elongate body 602 may represent a central flow portion configured to create and/or maintain an opening between two anatomical chambers. In some embodiments, the implant 600 may comprise multiple separate components which may be attached, connected, and/or otherwise joined to form a single device. For example, the elongate body 602 may comprise multiple components to form a generally tubular shape which may approximate a shape of an opening in a tissue wall.

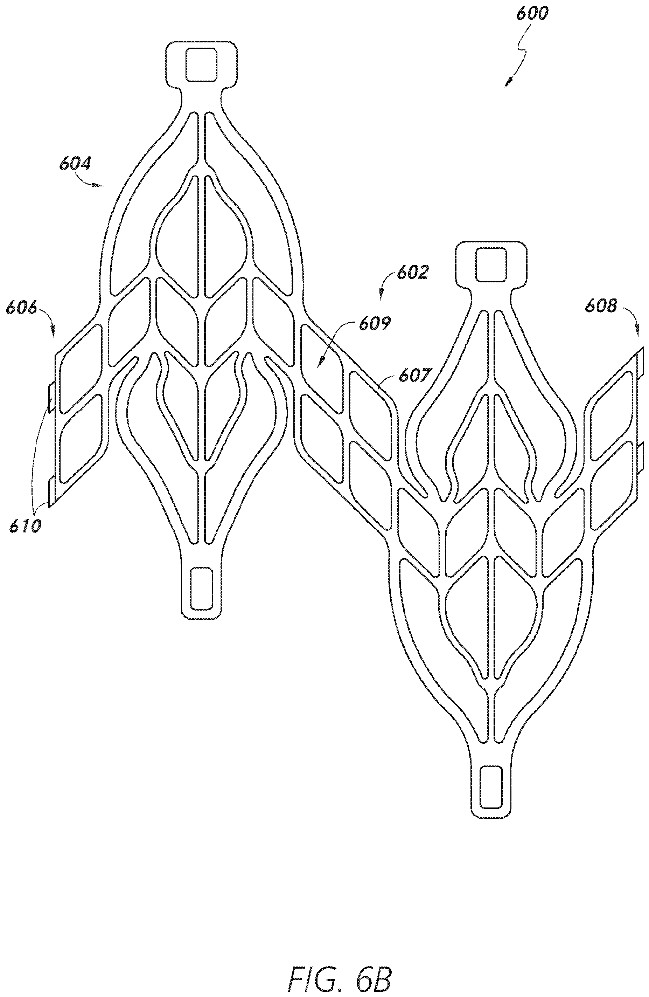

In some embodiments, the implant 600 may be configured to be movable between an expanded configuration and a collapsed (e.g., rolled and/or generally tubular) configuration to facilitate passage through a lumen of a catheter. For example, the elongate body 602 may be configured to be rolled, bent, twisted, or otherwise compacted to fit within the lumen of the catheter. The elongate body 602 may be configured to expand to a pre-defined shape (e.g., the shape and/or size shown in Figure 6A) and/or size during and/or after delivery within the body. The implant 600 further comprises one or more anchoring arms 604, which may include flanges, arms, anchors, and/or other devices. In some embodiments, the one or more anchoring arms 604 may be configured to extend generally perpendicularly (i.e., forming a "T" shape) from the elongate body. The one or more anchoring arms 604 may have a generally flat, curved, and/or wavy form. In some embodiments, the one or more anchoring arms 604 may be configured to at least partially collapse and/or compress to facilitate passage through the lumen of the catheter and/or may be configured to expand during and/or after delivery within the body to contact and/or attach to a tissue wall. Expansion of the implant 600 may be initiated, for example, by retraction of an outer sheath of the catheter relative to an inner support sheath. The implant 600 may be collapsed (e.g., rolled, spiraled) into a generally tubular configuration between the two sheaths with the anchoring arms 604 rolled, bent, and/or straightened. In some embodiments, the anchoring arms 604 may be configured to spring open when the restraining outer sheath retracts. The anchoring arms 604 may expand generally in opposite directions in a common plane to form a T-shape (see Figure 6B), as opposed to expanding in a circular fashion. Radiopaque markers on the anchoring arms 604 and/or elongate body 602 may be provided to facilitate positioning immediately within the body.

A pair of anchoring arms 604 (e.g., a first anchoring arm 604a and a second anchoring arm 604b) forms a clamping (i.e., pinching) pair of anchoring arms 604. The pairs of anchoring arms 604 are configured to apply a compressive force to a tissue wall to hold the implant 600 in place. The amount of compressive force may be relatively small to avoid damage to the tissue wall while sufficient to hold the implant 600 in place. For example, gaps separating the pairs of anchoring arms may be calibrated to avoid excessive clamping and/or necrosis of the tissue. The anchoring arms 604 may be configured to secure the implant 600 on generally opposite sides of the tissue wall and/or on generally opposite sides of an opening in the tissue wall. The elongate body 602 may be configured to be aligned generally perpendicular to the tissue wall so as to maintain an open flow path between the chambers on either side of the tissue wall. In alternative embodiments, the implant 600 may not comprise anchoring arms 604 and/or may be configured to be anchored through use of separating anchoring elements and/or through use of friction between the implant 600 and one or more tissue walls.

Components of the implant 600 may be configured to naturally self-expand due to inherent springiness and/or flexibility of the components. For example, various components (e.g., the elongate body 602 and/or anchoring arms 604) may be at least partially composed of an elastic material such as Nitinol. In some embodiments, the elongate body 602 may be fabricated by laser cutting a Nitinol tube.

As shown in Figures 6A-6C, the elongate body 602 may be composed of generally thin struts 607 in a generally parallelogram arrangement that may form an array of parallelogram-shaped cells 609 or openings. However, the elongate body 602, including the struts 607 and/or cells 609, may have any shape, size, and/or orientation. For example, the struts 607 may have a generally thin form such that various attachment mechanisms may be configured to latch onto and/or partially encircle the struts 607. However, the struts 607 may have a thicker design than shown in Figures 6A-6C to reduce and/or minimize the size of the cells 609. Rather than a generally parallelogram shape, the cells 609 may have a generally elliptical, triangular, hexagonal, or other shape. Moreover, the elongate body 602 may not comprise any cells 609. In some embodiments, the shape of the struts 607, cells 609, and/or the elongate body 602 generally may facilitate a collapsibility and/or expandability of the elongate body 602 for passage through a lumen of a catheter.

The elongate body 602 may be configured to form a generally tubular or other shape to approximate a shape of the opening. In some embodiments, the opening may be widened in all directions approximately evenly from a puncture point to form an approximately circular opening having a certain diameter. Accordingly, the elongate body 602, including the struts 607, may be configured to hold an at least partially rounded and/or circular form around/about the opening along a longitudinal axis (i.e., into the opening).

In some embodiments, the expandable implant 600 may be in a compacted and/or otherwise expandable form at delivery. For example, at delivery, the elongate body 602 and/or anchoring arms 604 may be folded, bent, and/or otherwise compacted to have a minimal profile to facilitate passage through a delivery catheter. After delivery, the elongate body 602 and/or anchoring arms 604 may be configured to unfold, unwrap, and/or otherwise expand (e.g., to form the design shown in Figure 6A). In some embodiments, at least a portion of the elongate body 602 and/or anchoring arms 604 may be composed of Nitinol and/or a similar material having shape-memory characteristics such that the implant 600 may naturally assume a pre-determined form after removal from the delivery catheter.

An implant 600 may comprise non-continuous components and/or may be configured to be cut and/or otherwise disconnected to form a non-continuous tube form. In some embodiments, the implant 600 may be configured to be cut and/or separated at the elongate body 602. A first cut line 611 and a second cut line 613 in Figure 6A represent example sections of the implant 600 that may be cut to from a discontinuous implant 600. The first cut line 611 provides an example in which the implant 600 may be cut along the struts 607 of the implant 600 and the second cut line 613 provides an example in which the implant 600 may be cut through the struts 607 along a line passing through one or more cells 609 of the implant 600. In some embodiments, a single cut may be made through the implant 600 and/or the implant 600 may be disconnected at a single point while in some embodiments, the implant 600 may be cut in multiple places and/or the implant 600 may be disconnected at multiple points.

In some embodiments, the implant 600 may be fully formed as shown in Figure 6A and/or one or more cuts may detach and/or create separations at points of the implant 600 without pre-existing cuts and/or separations. However, the implant 600 may be pre-cut and/or may comprise one or more discontinuous portions which may be configured to be attached and/or joined together to form the shape shown in Figure 6A and/or a similar shape. For example, the implant 600 may comprise one or more tabs and/or similar joining mechanisms at or near the first cut line 611, second cut line 613, and/or other portion(s) of the implant 600 which may be configured to interact with other features of the implant 600 to form a given shape (e.g., the form of the implant 600 shown in Figure 6A). In some embodiments, the implant 600 may be configured to be cut and/or otherwise detached to form one or more free ends prior to rolling and/or otherwise reducing a profile of the implant 600. After the one or more ends are detached and/or after the implant 600 is removed from a catheter and/or other delivery device, the one or more ends may be attached and/or reattached to form a continuous device.

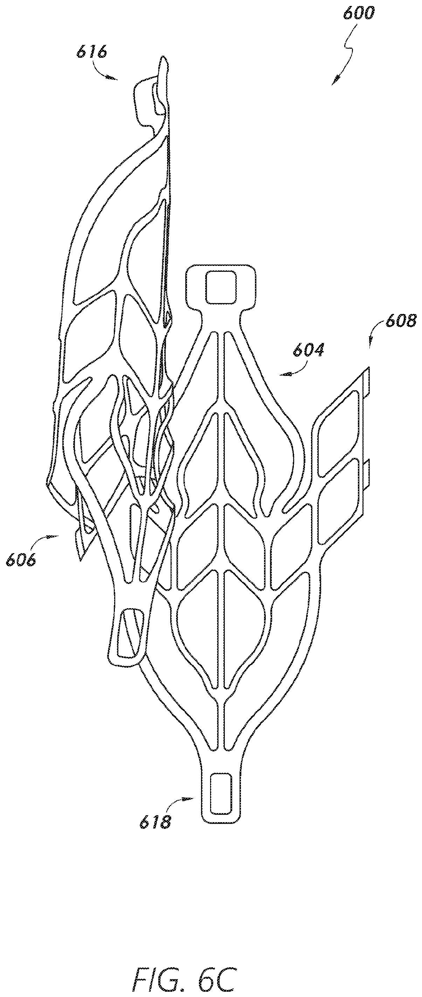

The elongate body/central flow portion 602 may be configured to define a flow patch between two anatomical chambers (e.g., the coronary sinus and the left atrium). For example, the elongate body 602 may be place within an opening in a tissue wall between the two anatomical chambers. The first end 606 and the second end 608 are configured to be alternately joined and/or detached. According to the present invention, one or more attachment mechanisms 610 at the first end 606 may be configured to alternately join to the second end 608 and/or attachment mechanisms 610 at the second end 608 and/or to detach from the second end 608 and/or attachment mechanisms 610 at the second end 608. Joining the first end 606 to the second end 608 may cause the elongate body 602 to form a generally tubular form (e.g., the form shown in Figure 6A), in which the elongate body 602 (i.e., central flow portion) may form a complete ellipse and/or cylinder about a flow path through a tissue wall. When the first end 606 is detached from the second end 608, the elongate body 602 may have a generally flat and/or partial tubular form (see, e.g., Figure 6B). Moreover, when the first end 606 is detached from the second end 608, the elongate body 602 and/or anchoring arms 604 are configured to be rolled (see, e.g., Figure 6C). In some embodiments, the anchoring arms 604 may be configured to extend from the elongate body 602 and/or anchor to a tissue wall separating two anatomical chambers.

In some embodiments, rolling the elongate body 602 may cause at least partial overlap of the elongate body 602. For example, the first end 606 may be rolled in-line with the second end 608 such that the first end 606 may pass over or under the second end 608. In some embodiments, rolling the elongate body 602 may cause no overlap of the elongate body 602. For example, the elongate body 602 may be spiraled such that a distance between the first end 606 and the second 608 increases during the rolling process (see, e.g., Figure 5B). In some embodiments, the anchoring arms 604 may be configured to be rolled (e.g., spiraled) to reduce the profile of the anchoring arms 604.

Figure 6B illustrates a cut and/or otherwise discontinuous implant 600 in a non-compressed form. The implant 600 comprises a first end 606 and/or a second end 608. In some embodiments, the first end 606 and/or the second end 608 may be formed by cutting the implant 600 shown in Figure 6A, for example at the first cut line 611. The implant 600 is shown in Figure 6B as having a generally flat form. In some embodiments, the implant 600 may be pre-formed (e.g., through use of a shape-memory alloy) to have a flat structure such that when the implant 600 is cut and/or otherwise forms the discontinuous form shown in Figure 6B, the implant 600 may be configured to naturally assume a generally flat form. For example, the implant 600 may naturally assume a form in which the first end 606 and the second end 608 are distal from each other and/or at opposite ends of the implant 600. In some embodiments, the implant 600 may be configured to naturally assume the form shown in Figure 6A. For example, the elongate body 602 may naturally form a generally tubular form. Moreover, the first end 606 and the second end 608 may naturally assume a position in which the first end 606 and the second end 608 are in close proximity and/or in contact with each other. The anchoring arms 604 may be configured to naturally assume the generally flat form shown in Figure 6B or may naturally assume the at least partially curved form shown in Figure 6A.

In some embodiments, the implant 600 may comprise one or more attachment mechanisms 610 configured to be joined with other attachment mechanisms at the second end 608 and/or other portions of the implant 600. For example, attachment mechanisms 610 at or near the first end 606 may be configured to be engaged to attach to and/or otherwise contact the second end 608 and/or attachment mechanism 610 at or near the second end 608. An attachment mechanism 610 may include a tab, hook, loop, notch, peg, magnet, strap, pin, hole, socket, and/or other mechanism configured to join separate portions of the implant 600. In some embodiments, multiple attachment mechanisms 610 may be configured to be joined together. For example, a first attachment mechanism 610 (e.g., at the first end 606) may comprise a tab configured to fit into and/or join with a second attachment mechanism 610 (e.g., at the second end 608) that may comprise a notch configured to receive the hook. In some embodiments, the first end 606 may be configured to attach to and/or detach from the second end 608 through use of one or more attachment mechanisms 610. For example, the first end 606 may comprise a tab and/or other attachment mechanism 610 configured to be fit into and/or removed from an opening and/or feature at the second end 608. In some embodiments, the implant 600 detaching the first end 606 from the second end 608 may involve cutting the implant 600 and/or disengaging one or more attachment mechanisms 610. Engaging an attachment mechanism 610 may involve creating a secure attachment between the first end 606 and the second end 608. For example, a hook at the first end 606 may be fit into a corresponding loop at the second end 608. Similarly, disengaging an attachment mechanism 610 may involve breaking a secure attachment between the first end 606 and the second end 608. For example, a hook at the first end 606 may be removed from a corresponding loop at the second end 608.

While Figure 6B shows two attachment mechanisms 610 at the first end 606 and two attachment mechanisms 610 at the second end 608, the implant 600 may comprise any number of attachment mechanisms 610, including any number of attachment mechanisms 610 at or near the first end 606 and/or any number of attachment mechanisms 610 at or near the second end 608. Moreover, the implant 600 may comprise one or more notches, cavities, grooves, holes, and/or other features configured to receive attachment mechanisms 610. For example, one or more struts 607 of the implant 600 (e.g., at the first end 606 and/or second end 608) may comprise one or more cavities configured to receive tabs and/or other attachment mechanisms 610 of the implant 600. In some embodiments, the implant may comprise one or more attachment mechanisms 610 configured to pass at least partially through one or more cells 609 and/or other openings of the implant 600. For example, an attachment mechanism 610 may comprise a hook configured to pass at least partially through a cell 609 and/or hook onto one or more struts 607 of the implant 600 to hold the attachment mechanism 610 in place and/or to create a secure attachment between portions of the implant. In some embodiments, the first end 606 may comprise one or more attachment mechanism 610 (e.g., hooks, latches, fingers, etc.) configured to attach to and/or partially encircle one or more struts 607 at the second end 608 and/or the second end 608 may comprise one or more attachment mechanism 610 (e.g., hooks, latches, fingers, etc.) configured to attach to and/or partially encircle one or more struts 607 at the first end 606. A strut 607 may comprise a generally thin wire-like form.

In some embodiments, the implant 600 may be configured to be twisted, rolled, and/or spiraled to minimize the profile of the implant 600 during delivery. As shown in Figure 6C, at least some components of the implant 600 may be rolled and/or spiraled. In some embodiments, rolling and/or spiraling the implant 600 may involve bending and/or curving at least some portions of the implant 600 such that the first end 606 of the implant 600 is pressed against another portion of the implant 600. The implant 600 may be rolled until all portions of the implant 600 are bent and/or curved to some extent. Rolling and/or spiraling the implant 600 may involve first bending and/or curving the implant at or near the first end 606 and bending and/or curving the implant at or near the second end 608 after all other portions of the implant 600 are bent and/or curved to some extent.

As shown in Figure 6C, the implant 600 may be rolled laterally, starting with the first end 606 and ending with the second end 608. In this way, the first end 606 may be pressed against an anchoring arm 604 extending from the first end 606. When the implant 600 is rolled laterally, the length of the rolled implant 600 may be equal to a distance between a first end portion 616 of a first anchoring arm and a second end portion 618 of a second anchoring arm 604. The first end portion 616 and the second end portion 618 may be on opposite sides of the elongate body 602. For example, when the implant 600 is placed on a tissue wall (with each of the anchoring arms 604 pinching the tissue wall), the first end portion 616 may be configured to be placed at a first side of the tissue wall and/or within a first anatomical chamber and the second end portion 618 may be configured to be placed at a second side of the tissue wall and/or within a second anatomical chamber.

Additionally or alternatively, the implant 600 may be configured to be rolled longitudinally, starting with the first end portion 616 or the second end portion 618 and ending with the second end portion 618 or the first end portion 616, respectively. The first end portion 616 and/or second end portion 618 may be pressed against the anchoring arms 604. When the implant 600 is rolled longitudinally, the length of the rolled implant 600 may be equal to a distance between the first end 606 and the second end 608.

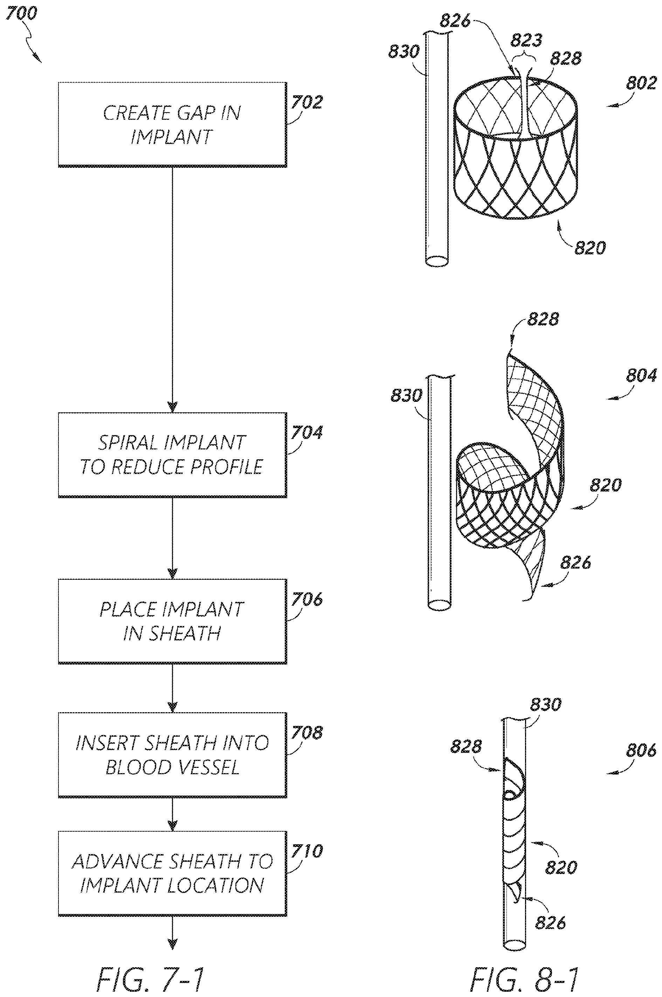

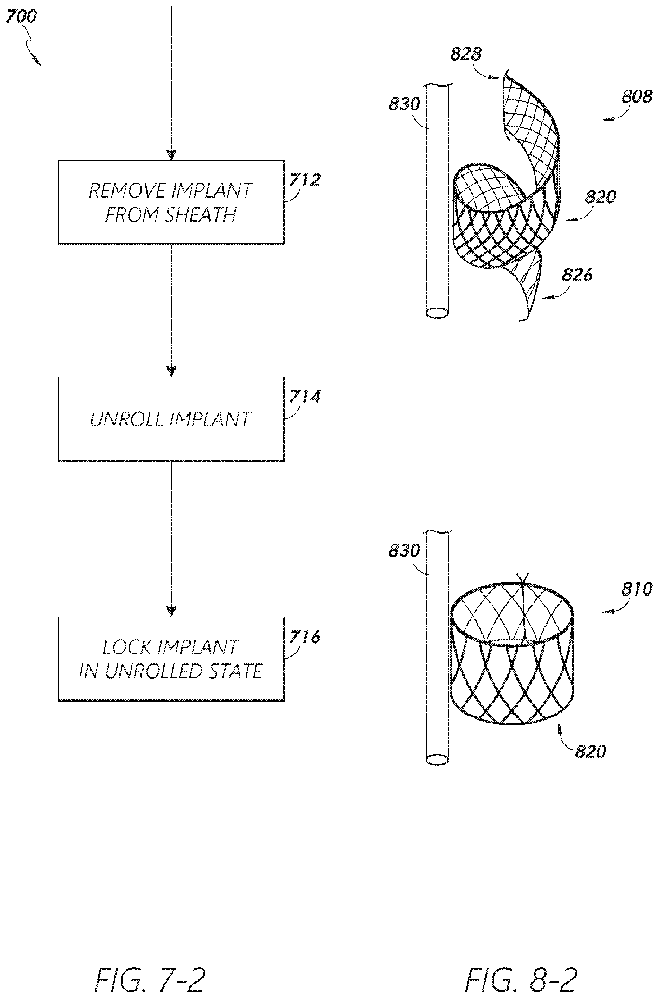

Figures 7-1 and 7-2 are a flow diagram illustrating a process 700 for rolling, spiraling, and/or twisting an implant to minimize the profile of the implant during delivery to a treatment site in accordance with one or more embodiments of the present disclosure. Figures 8-1 and 8-2 show an example implant 820 associated with the process 700 of Figures 7-1 and 7-2 to illustrate aspects of the process 700 according to one or more implementations thereof.

At block 702, the process 700 involves creating a separation 823 in the implant 800. In some embodiments, the implant 820 may be pre-formed to have multiple ends having a separation between the multiple ends, or the implant 820 may have an otherwise non-continuous form. As shown in the image 802, the separation 823 may represent a disconnect between a first end 826 and a second end 828 of the implant 820. The first end 826 and the second end 828 may be configured to be attached and/or joined to form a continuous implant 820. In some embodiments, creating the separation 823 may involve detaching multiple ends of the implant 820 by cutting along a continuous portion of the implant. However, creating the separation 823 may involve detaching one or more attachment mechanisms of the implant 820. For example, the first end 826 of the implant 820 may comprise one or more attachment mechanisms configured to mate with one or more attachment mechanisms and/or other features at the second end 828 of the implant 820. After the attachment mechanisms are disconnected, one or more attachment mechanisms may be joined together, for example following delivery at a treatment site within a body.

The implant 820 may have a generally cylindrical form prior to creation of the separation. After creating the separation, the implant 820 may generally maintain the cylindrical form and/or may unroll to form a generally flat device. In some embodiments, the implant 820 may be at least partially composed of a shape-memory alloy (e.g., Nitinol) and/or may otherwise be configured to be formed to any pre-defined shape. The implant 820 may comprise a mesh and/or other pattern of struts and/or wires having a generally thin form.

In some embodiments, an implant 820 may be configured to spiraled, twisted, rolled, and/or otherwise compressed without a separation 823 in the implant 820. Some components of an implant 820 may be configured to be bent, collapsed, and/or otherwise formed to allow other portions of the implant 820 to be rolled, spiraled, and/or twisted to reduce the overall profile of the implant 820. For example, one or more portions of the implant 820 may be composed a generally flexible material such that the one or more portions may be shaped as necessary to allow for rolling, spiraling, and/or twisting at least some portions of the implant 820.

At block 704, the process 700 involves spiraling and/or rolling the implant 820 to reduce the profile of the implant 820. As shown in image 804, spiraling the implant 820 may involve twisting the implant 820 to increase a distance between the first end 826 and the second end 828. When the implant 820 is spiraled, the implant 820 may form a generally tubular and/or cylindrical sheath around a hollow interior area. As the implant 820 is spiraled, the hollow interior area may decrease in volume and/or portions of the implant 820 may move closer together. An amount of spiraling and/or rolling of the implant 820 may be determined based on a size of a delivery catheter 830 configured to receive the implant 820. As shown in image 806, the implant 820 may be spiraled and/or rolled until a diameter and/or width of the implant 820 is approximately equal to or less than the dimeter and/or width of the delivery catheter 830. The terms "catheter," "delivery catheter," "sheath," and "means for delivery" are used herein according to their broad and ordinary meanings and may refer to any type of tube suitable for insertion in the body. "Catheter" and "sheath" may be used substantially interchangeably is some contexts herein.

While the catheter 830 is shown in Figures 8-1 and 8-2 without an inner support shaft (see, e.g., Figures 3A and 3B), the catheter 830 may include an inner support shaft within an outer tube of the catheter. The inner support shaft may be configured to facilitate rolling and/or exposing of the implant by pressing against the implant 820 and/or providing support to an inner surface of the implant 820.

At block 706, the process 700 involves placing the implant 820 into the delivery catheter 830. In some embodiments, the entire implant 820 may be placed into the catheter 830. However, only a portion of the implant 820 may be placed into the catheter 830 in some embodiments. While within the catheter 830, the catheter 830 may prevent expansion of the implant 820. For example, the implant 820 may be configured to naturally expand and/or naturally return to a pre-defined form that has a greater diameter/width than the catheter 830. However, the catheter 830 may be configured to prevent such expansion of the implant 820.

At block 708, the process 700 involves inserting the catheter 830 containing the implant 820 into a body of a patient. In some embodiments, the catheter 830 may be inserted into a blood vessel (e.g., the coronary sinus). At block 710, the process 700 involves advancing the sheath through the body to a desired implant location. The implant 820 may remain at least partially within the catheter 830 as the catheter 830 moves through the body.

At block 712, the process 700 involves removing the implant 820 from the catheter 830 when the catheter 830 is delivered to the implant location. In some embodiments, removing the implant 820 may involve pulling back at least a portion of the catheter 830 to expose the implant 820 to the blood vessel and/or other portion of the body. When the implant 820 is exposed from the catheter 830, the implant 820 may at least partially unroll and/or otherwise expand. At block 714, the process 700 involves unrolling and/or otherwise expanding the implant 820. As shown in image 808, the implant 820 may generally reverse the rolling and/or spiraling process performed to place the implant 820 into the catheter 830.

In some embodiments, the implant 820 may be configured to naturally unroll and/or otherwise expand upon being at least partially removed from the catheter 830. For example, removing the implant 820 may cause unrolling of the implant 820 to an expanded profile. This may advantageously allow surgeons to more easily deploy the implant 820 following delivery through the catheter 830. However, unrolling and/or expansion of the implant 820 may be assisted at least in part. For example, the catheter 830 and/or another surgical tool may be used to press against and/or pull the implant 820 to move the implant 820 towards an expanded shape and/or position.

At block 716, the process 700 involves locking the implant 820 in an unrolled and/or expanded state shown in image 810. In some embodiments, the implant 820 may be locked in position at least in part by virtue of shape-memory characteristics of the implant 820. For example, the implant 820 may be at least partially composed of Nitinol and/or may be predisposed to hold the unrolled and/or expanded form. The expanded state of the implant 820 may be identical or similar to the pre-spiraled state shown in image 802. However, the implant 820 may be at least partially flat in the expanded state. For example, the implant 820 may naturally form a generally flat device as shown in Figure 6B.

In some embodiments, one or more locking and/or attachment mechanisms may be used to lock the implant 820 in place and/or otherwise provide compressive resistance to the implant 820. The one or more locking and/or attachment mechanisms may extend from and/or attach to the implant 820 and/or may be separate devices from the implant 820.

### Additional Embodiments

Depending on the embodiment, certain acts, events, or functions of any of the processes or algorithms described herein can be performed in a different sequence, may be added, merged, or left out altogether. Thus, in certain embodiments, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

It should be appreciated that in the above description of embodiments, various features are sometimes grouped together in a single embodiment, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular embodiment herein can be applied to or used with any other embodiment(s). Further, no component, feature, step, or group of components, features, or steps are necessary or indispensable for each embodiment. Thus, it is intended that the scope of the inventions herein disclosed and claimed below should not be limited by the particular embodiments described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). **In** addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example embodiments belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

Thus, the scope of the claims that may arise herefrom is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

Delivery systems as described herein may be used to position catheter tips and/or catheters to various areas of a human heart. For example, a catheter tip and/or catheter may be configured to pass from the right atrium into the coronary sinus. However, it will be understood that the description can refer or generally apply to positioning of catheter tips and/or catheters from a first body chamber or lumen into a second body chamber or lumen, where the catheter tips and/or catheters may be bent when positioned from the first body chamber or lumen into the second body chamber or lumen. A body chamber or lumen can refer to any one of a number of fluid channels, blood vessels, and/or organ chambers (e.g., heart chambers). Additionally, reference herein to "catheters," "tubes," "sheaths," "steerable sheaths," and/or "steerable catheters" can refer or apply generally to any type of elongate tubular delivery device comprising an inner lumen configured to slidably receive instrumentation, such as for positioning within an atrium or coronary sinus, including for example delivery catheters and/or cannulas. It will be understood that other types of medical implant devices and/or procedures can be delivered to the coronary sinus using a delivery system as described herein, including for example ablation procedures, drug delivery and/or placement of coronary sinus leads.

## Claims

1. A medical implant (402; 600) comprising an elongate body having a first end (406; 606), a second end (408; 608) and two or more anchoring arms (604) extending from the elongate body, wherein the elongate body is configured to be rolled to a reduced profile, and to fit into a catheter (404) in the reduced profile; the elongate body being further configured to unroll to an expanded profile in response to being removed from the catheter (404); wherein the two or more anchoring arms (604) are configured to anchor to one or more tissue walls, wherein at least one pair of anchoring arms (604) is configured to apply a compressive force therebetween to a tissue wall to hold the medical implant (402; 600) in place; **characterized in that** the first end comprises one or more attachment mechanisms (610) configured to alternately join with the second end (408; 608) in the expanded profile of the elongate body and detach from the second end (408; 608) for being rolled to the reduced profile of the elongate body.

2. The medical implant (402; 600) of claim 1, wherein the elongate body is at least partially composed of a shape-memory alloy.

3. The medical implant (402; 600) of any of claims 1 or 2, wherein the elongate body is configured to fit at least partially within an opening in a tissue wall and providing a blood flow path between a first anatomical chamber and a second anatomical chamber, and wherein the elongate body is configured to maintain the blood flow path from the first anatomical chamber to the second anatomical chamber.

4. The medical implant (402; 600) of any of claims 1 to 3, wherein rolling the elongate body portion causes at least partial overlap of the elongate body.

5. The medical implant (402; 600) of any of claims 1 to 3, wherein rolling the elongate body causes no overlap of the elongate body and increases a distance between the first end (406; 606) and the second end (408; 608).

6. The medical implant (402; 600) of claim 2, wherein the two or more anchoring arms (604) are configured to be rolled.

7. The medical implant (402; 600) of any of claims 1 to 6, wherein the elongate body is configured to naturally unroll in response to being removed from the catheter.

8. The medical implant (402; 600) of any of claims 1 to 7, wherein the one or more attachment mechanisms (610) comprise one or more of a tab, a hook, a loop, a notch, a peg, a magnet, a strap, a pin, a hole and/or a socket.

9. The medical implant (402; 600) of any of claims 1 to 8, wherein the second end (408) comprises one or more attachment mechanisms (610), wherein one of the one or more attachment mechanisms at the first end (406; 606) is configured to be joined with one of the one or more attachment mechanisms at the second end (408; 608).

10. The medical implant (402; 600) of claim 9, wherein the one of the one or more attachment mechanisms at the first end (406; 606) comprises a tab and the one of the one or more attachment mechanisms at the second end (408; 608) comprises a notch, wherein the tab is configured to fit into and/or join with the notch.

11. The medical implant (402; 600) of claim 9, wherein the one of the one or more attachment mechanisms (610) at the first end (406; 606) comprises a hook and the one of the one or more attachment mechanisms (610) at the second end (408; 608) comprises a loop, wherein the hook is configured to fit into the loop.

12. The medical implant (402; 600) of any of claims 1 to 8, further comprising one or more attachment mechanisms (610) configured to pass at least partially through one or more cells (609) of the implant (600).

13. The medical implant (402; 600) of claim 12, wherein the one of the one or more attachment mechanisms (610) comprises a hook configured to pass at least partially through the cell (609).

14. The medical implant (402; 600) of claim 9; wherein the one or more attachment mechanisms (610) comprises a hook configured to hook into one or more struts (607) of the implant (402; 600) to create secure attachment between portions of the implant.

15. The medical implant (402; 600) of any of claims 1 to 12, further comprising one or more notches, cavities, grooves and/or holes configured to receive the one or more attachment mechanisms (610).

## Patentansprüche

1. Medizinisches Implantat (402; 600), das einen länglichen Körper umfasst, der ein erstes Ende (406; 606) und ein zweites Ende (408; 608) und zwei oder mehr Verankerungsarme (604) aufweist, die sich von dem länglichen Körper erstrecken, wobei der längliche Körper ausgebildet ist, in ein reduziertes Profil gerollt zu werden und in dem reduzierten Profil in einen Katheter (404) zu passen; wobei der längliche Körper weiter ausgebildet ist, sich zu einem expandierten Profil auszurollen, als Reaktion darauf, aus dem Katheter (404) entfernt zu werden;
wobei die zwei oder mehr Verankerungsarme (604) ausgebildet sind, sich an einer oder mehreren Gewebewänden zu verankern, wobei mindestens ein Paar der Verankerungsarme (604) ausgebildet ist, eine Druckkraft zwischen sich auf eine Gewebewand auszuüben, um das medizinische Implantat (402; 600) an der Stelle zu halten;
**dadurch gekennzeichnet, dass** das erste Ende einen oder mehrere Anbringungsmechanismen (610) umfasst, die ausgebildet sind, sich abwechselnd mit dem zweiten Ende (408; 608) im expandierten Profil des länglichen Körpers zu verbinden und sich vom zweiten Ende (408; 608) zu lösen, um zu dem reduzierten Profil des länglichen Körpers gerollt zu werden.

2. Medizinisches Implantat (402; 600) nach Anspruch 1, wobei der längliche Körper zumindest teilweise aus einer Formgedächtnislegierung besteht.

3. Medizinisches Implantat (402; 600) nach einem der Ansprüche 1 oder 2, wobei der längliche Körper ausgebildet ist, zumindest teilweise in eine Öffnung in einer Gewebewand zu passen und einen Blutflussweg zwischen einer ersten anatomischen Kammer und einer zweiten anatomischen Kammer bereitzustellen, und wobei der längliche Körper ausgebildet ist, den Blutflussweg von der ersten anatomischen Kammer zu der zweiten anatomischen Kammer aufrechtzuerhalten.

4. Medizinisches Implantat (402; 600) nach einem der Ansprüche 1 bis 3, wobei Rollen des länglichen Körperabschnitts eine zumindest teilweise Überlappung des länglichen Körpers bewirkt.

5. Medizinisches Implantat (402; 600) nach einem der Ansprüche 1 bis 3, wobei Rollen des länglichen Körpers keine Überlappung des länglichen Körpers bewirkt und einen Abstand zwischen dem ersten Ende (406; 606) und dem zweiten Ende (408; 608) vergrößert.

6. Medizinisches Implantat (402; 600) nach Anspruch 2, wobei die zwei oder mehr Verankerungsarme (604) ausgebildet sind, um gerollt zu werden.

7. Medizinisches Implantat (402; 600) nach einem der Ansprüche 1 bis 6, wobei der längliche Körper ausgebildet ist, sich auf natürliche Weise auszurollen, als Reaktion darauf, dass er aus dem Katheter entfernt wird.

8. Medizinisches Implantat (402; 600) nach einem der Ansprüche 1 bis 7, wobei der eine oder die mehreren Anbringungsmechanismen (610) eine oder mehrere Laschen, Haken, Schlaufen, Kerben, Stifte, Magnete, Riemen, Pins, Löcher und/oder Buchsen umfassen.

9. Medizinisches Implantat (402; 600) nach einem der Ansprüche 1 bis 8, wobei das zweite Ende (408) einen oder mehrere Anbringungsmechanismen (610) umfasst, wobei einer der einen oder mehreren Anbringungsmechanismen an dem ersten Ende (406; 606) ausgebildet ist, mit einem der einen oder mehreren Anbringungsmechanismen an dem zweiten Ende (408; 608) verbunden zu sein.

10. Medizinisches Implantat (402; 600) nach Anspruch 9, wobei der eine der einen oder mehreren Anbringungsmechanismen an dem ersten Ende (406; 606) eine Lasche umfasst und der eine der einen oder mehreren Anbringungsmechanismen an dem zweiten Ende (408; 608) eine Kerbe umfasst, wobei die Lasche ausgebildet ist, in die Kerbe zu passen und/oder mit dieser verbunden zu sein.

11. Medizinisches Implantat (402; 600) nach Anspruch 9, wobei der eine der einen oder mehreren Anbringungsmechanismen (610) am ersten Ende (406; 606) einen Haken umfasst und der eine der einen oder mehreren Anbringungsmechanismen (610) am zweiten Ende (408; 608) eine Schlaufe umfasst, wobei der Haken ausgebildet ist, in die Schlaufe zu passen.

12. Medizinisches Implantat (402; 600) nach einem der Ansprüche 1 bis 8, das weiter einen oder mehrere Anbringungsmechanismen (610) umfasst, die ausgebildet sind, zumindest teilweise durch eine oder mehrere Zellen (609) des Implantats (600) hindurch zu reichen.

13. Medizinisches Implantat (402; 600) nach Anspruch 12, wobei der eine der einen oder mehreren Anbringungsmechanismen (610) einen Haken umfasst, der ausgebildet ist, zumindest teilweise durch die Zelle (609) hindurch zu reichen.

14. Medizinisches Implantat (402; 600) nach Anspruch 9, wobei der eine oder die mehreren Anbringungsmechanismen (610) einen Haken umfasst, der ausgebildet ist, in eine oder mehrere Streben (607) des Implantats (402; 600) einzuhaken, um eine sichere Anbringung zwischen Abschnitten des Implantats zu erzeugen.

15. Medizinisches Implantat (402; 600) nach einem der Ansprüche 1 bis 12, das weiter eine oder mehrere Kerben, Vertiefungen, Rillen und/oder Löcher umfasst, die ausgebildet sind, um den einen oder die mehreren Anbringungsmechanismen (610) aufzunehmen.

## Revendications

1. Implant médical (402 ; 600) comprenant un corps allongé présentant une première extrémité (406 ; 606), une seconde extrémité (408 ; 608) et deux bras d'ancrage (604) ou plus s'étendant à partir du corps allongé, le corps allongé étant conçu pour être enroulé en un profilé réduit et pour s'ajuster dans un cathéter (404) dans le profilé réduit ; le corps allongé étant en outre conçu pour se dérouler vers un profilé déployé en réponse à son retrait du cathéter (404) ; les deux bras d'ancrage (604) ou plus étant conçus pour s'ancrer à une ou plusieurs parois tissulaires, au moins une paire de bras d'ancrage (604) étant conçue pour appliquer une force de compression entre eux sur une paroi tissulaire afin de maintenir l'implant médical (402 ; 600) en place ;
**caractérisé en ce que** la première extrémité comprend un ou plusieurs mécanismes d'attache (610) conçus pour se joindre en alternance à la seconde extrémité (408 ; 608) dans le profilé déployé du corps allongé et se détacher de la seconde extrémité (408 ; 608) pour être enroulés en profilé réduit du corps allongé.

2. Implant médical (402 ; 600) selon la revendication 1, le corps allongé étant au moins partiellement composé d'un alliage à mémoire de forme.

3. Implant médical (402 ; 600) selon l'une quelconque des revendications 1 ou 2, le corps allongé étant conçu pour s'ajuster au moins partiellement à l'intérieur d'une ouverture dans une paroi tissulaire et pour fournir un trajet d'écoulement sanguin entre une première chambre anatomique et une seconde chambre anatomique et le corps allongé étant conçu pour maintenir le trajet d'écoulement sanguin à partir de la première chambre anatomique vers la seconde chambre anatomique.

4. Implant médical (402 ; 600) selon l'une quelconque des revendications 1 à 3, l'enroulement de la partie de corps allongé provoquant un chevauchement au moins partiel du corps allongé.

5. Implant médical (402 ; 600) selon l'une quelconque des revendications 1 à 3, l'enroulement du corps allongé ne provoquant aucun chevauchement du corps allongé et augmentant une distance entre la première extrémité (406 ; 606) et la seconde extrémité (408 ; 608).

6. Implant médical (402 ; 600) selon la revendication 2, les deux bras d'ancrage (604) ou plus étant conçus pour être enroulés.

7. Implant médical (402 ; 600) selon l'une quelconque des revendications 1 à 6, le corps allongé étant conçu pour se dérouler naturellement en réponse à son retrait du cathéter.

8. Implant médical (402 ; 600) selon l'une quelconque des revendications 1 à 7, ledit un ou lesdits plusieurs mécanismes d'attache (610) comprenant un ou plusieurs parmi une languette, un crochet, une boucle, une encoche, un tenon, un aimant, une sangle, une broche, un trou et/ou une emboîture.

9. Implant médical (402 ; 600) selon l'une quelconque des revendications 1 à 8, la seconde extrémité (408) comprenant un ou plusieurs mécanismes d'attache (610), un mécanisme parmi ledit un ou lesdits plusieurs mécanismes d'attache au niveau de la première extrémité (406 ; 606) étant conçu pour être joint à un mécanisme parmi ledit un ou lesdits plusieurs mécanismes d'attache au niveau de la seconde extrémité (408 ; 608).

10. Implant médical (402 ; 600) selon la revendication 9, ledit un mécanisme parmi ledit un ou lesdits plusieurs mécanismes d'attache au niveau de la première extrémité (406 ; 606) comprenant une languette et ledit un mécanisme parmi ledit un ou lesdits plusieurs mécanismes d'attache au niveau de la seconde extrémité (408 ; 608) comprenant une encoche, la languette étant conçue pour s'ajuster dans et/ou se joindre à l'encoche.

11. Implant médical (402 ; 600) selon la revendication 9, ledit un mécanisme parmi ledit un ou lesdits plusieurs mécanismes d'attache (610) au niveau de la première extrémité (406 ; 606) comprenant un crochet et ledit un mécanisme parmi ledit un ou lesdits plusieurs mécanismes d'attache (610) au niveau de la seconde extrémité (408 ; 608) comprenant une boucle, le crochet étant conçu pour s'ajuster dans la boucle.

12. Implant médical (402 ; 600) selon l'une quelconque des revendications 1 à 8, comprenant en outre un ou plusieurs mécanismes d'attache (610) conçus pour passer au moins partiellement à travers une ou plusieurs cellules (609) de l'implant (600).

13. Implant médical (402 ; 600) selon la revendication 12, ledit un mécanisme parmi ledit un ou lesdits plusieurs mécanismes d'attache (610) comprenant un crochet conçu pour passer au moins partiellement à travers la cellule (609).

14. Implant médical (402 ; 600) selon la revendication 9, ledit un ou lesdits plusieurs mécanismes d'attache (610) comprenant un crochet conçu pour s'accrocher dans une ou plusieurs entretoises (607) de l'implant (402 ; 600) afin de créer une attache sûre entre des parties de l'implant.

15. Implant médical (402 ; 600) selon l'une quelconque des revendications 1 à 12, comprenant en outre un(e) ou plusieurs encoche(s), cavité(s), rainure(s) et/ou trou(s) conçu(e)(s) pour recevoir ledit un ou lesdits plusieurs mécanismes d'attache (610).
